(19) Europäisches Patentamt
European Patent Office
Office européen des brevets

(11) **EP 3 029 587 A1**

(12) **EUROPÄISCHE PATENTANMELDUNG**

(43) Veröffentlichungstag:
**08.06.2016 Patentblatt 2016/23**

(51) Int Cl.:
***G06F 19/00*** *(2011.01)* ***A63B 24/00*** *(2006.01)*

(21) Anmeldenummer: **14196420.5**

(22) Anmeldetag: **04.12.2014**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Pulse7 GmbH**
**4050 Traun (AT)**

(72) Erfinder: **Konrad, Martin**
**4614 Marchtrenk (AT)**

(74) Vertreter: **Jell, Friedrich et al**
**Bismarckstrasse 9**
**4020 Linz (AT)**

(54) **Verfahren zum Erzeugen einer Trainingsanweisung für ein körperliches Training**

(57) Es wird ein Verfahren zum Erzeugen einer Anweisung für ein körperliches Training gezeigt, bei dem die Herzratenvariabilität (HRV) und/oder die Herzfrequenz (HF) des zu Trainierenden erfasst und in deren Abhängigkeit dessen physischer und/oder psychischer Zustand bestimmt und anhand dieses Zustands für diesen eine Anweisung zu einem bevorstehenden Training erzeugt wird, die eine Empfehlung zu mindestens einem Belastungsbereich von körperlichen Belastungsintensi-täten umfasst. Um ein besonders einfaches, sicheres und zuverlässiges Verfahren zur Verfügung zu stellen, wird vorgeschlagen, dass anhand des am Tag des Trainings bestimmten aktuellen physischen und/oder psychischen Zustands mindestens ein Belastungsbereich für das Training an diesem Tag aus, dem zu Trainierenden zuvor individuell vorgegebenen, unterschiedlichen Belastungsbreichen ausgewählt und zum Training empfohlen wird.

EP 3 029 587 A1

**Beschreibung**

Die Erfindung betrifft ein Verfahren zum Erzeugen einer Anweisung für ein körperliches Training, bei dem die Herzratenvariabilität (HRV) und/oder die Herzfrequenz (HF) des zu Trainierenden erfasst und in deren Abhängigkeit dessen physischer und/oder psychischer Zustand bestimmt und anhand dieses Zustands für diesen eine Anweisung zu einem bevorstehenden Training erzeugt wird, die eine Empfehlung zu mindestens einem Belastungsbereich von körperlichen Belastungsintensitäten umfasst.

Aus dem Stand der Technik sind Verfahren bekannt, um Anweisungen für ein körperliches Training zu erzeugen. Dies erfolgt etwa (EP1852062B1) auf Basis des Vergleichs von, unter Standardzuständen erfassten, HRV-Werten des zu Trainierenden mit einem Referenzwert, welcher aus einer Vielzahl zuvor aufgezeichneter HRV-Werte des zu Trainierenden ermittelt wurde, zu erzeugen. Auf diese Weise erzeugte Anweisungen können unter anderem Anweisungen zur Intensität des Trainings, also zur Belastungsintensität, umfassen. Die Standardzustände können etwa bestimmte Tageszeitpunkte sein (z. B. nach dem Aufstehen) oder einer definierten physischen Aktivität zugeordnet sein (ruhend, locker laufend, etc.). Eine derartige Anweisung kann jedoch den aktuellen physischen und/oder physische Zustand des Trainierenden - und zwar insbesondere dessen Erholungszustand - nur sehr eingeschränkt berücksichtigen. Dies wirkt sich auch entsprechend nachteilig auf den Trainingseffekt aus - ein derartiges Verfahren zeigt somit eine geringe Zuverlässigkeit bei der Empfehlung von, zur Erreichung dieses Trainingsziels, optimalen Belastungsintensitäten. Nicht zuletzt besteht die Gefahr, dass für ein bevorstehendes Training zu intensive oder zu leichte Belastungsintensitäten empfohlen werden, was etwa zu Übertraining oder einer Unterforderung des zu Trainierenden führen kann.

Weiter ist bekannt, die Belastungsintensitäten des körperlichen Trainings, individuell für den Trainierenden, in verschiedene Belastungsbereiche zu klassifizieren (EP2608090A1, EP2260910A1). Die Belastungsbereiche können dabei im einfachsten Fall über Herzfrequenzbereiche definiert werden, die Belastungsintensitäten umfassen kann. Solche Verfahren dienen allerdings lediglich der Klassifizierung der Belastungsintensitäten vergangener Trainings oder eines laufenden Trainings. Für die Erzeugung einer Anweisung zu einem bevorstehenden Training oder für ein, in Bezug auf den physischen und/oder psychischen Zustand des zu Trainiereden optimiertes Training sind derartige Verfahren nicht geeignet.

Die Erfindung hat sich daher die Aufgabe gestellt, ausgehend vom eingangs geschilderten Stand der Technik ein besonders einfaches und dennoch zuverlässiges sowie sicheres Verfahren zur Verfügung zu stellen, anhand welchem optimale Trainingsergebnisse erreichbar sind. Des Weiteren sollen gute Steuerbarkeit und Variabilität des Verfahrens sichergestellt werden.

Die Erfindung löst die gestellte Aufgabe dadurch, dass anhand des am Tag des Trainings bestimmten aktuellen physischen und/oder psychischen Zustands mindestens ein Belastungsbereich für das Training an diesem Tag aus, dem zu Trainierenden zuvor individuell vorgegebenen, unterschiedlichen Belastungsbreichen ausgewählt und zum Training empfohlen wird.

Wird anhand des am Tag des Trainings aktuellen physischen und/oder psychischen Zustands mindestens ein Belastungsbereich für das Training an diesem Tag ausgewählt, kann zunächst die aktuelle Verfassung des zu Trainierenden im erfindungsgemäßen Verfahren besonders gut Berücksichtigung finden. Es ist also möglich, die Auswirkungen eines vorangegangenen körperlichen Trainings, diverse Lebensumstände wie Ernährung, Schlafqualität usw. verbessert in ein bevorstehendes Training einfließen zu lassen - womit für diesen in weiterer Folge auch die Optimierung einer individuellen Anweisung zu einem bevorstehenden Training hinsichtlich Effektivität zu erreichender Trainingsziele etc. sichergestellt werden kann.

Dadurch, dass erfindungsgemäß zudem mindestens ein Belastungsbereich für das Training an diesem Tag ausgewählt wird, kann das Training unter anderem vergleichsweise variabel gestaltet und verbessert gesteuert werden. Bei Bedarf können also auch mehrere Belastungsbereiche gewählt und das Training auf den zu Trainierenden individuell besonders gut abgestimmt werden. Es ist somit etwa möglich, verschiedene Belastungsbereiche und somit unterschiedliche Belastungsintensitäten und/oder -dauern für ein bevorstehendes Training zu kombinieren - womit das Training entsprechend dem aktuellen physischen und/oder psychischen Zustand des zu Trainierenden besonders gut auf das zu erreichenden Trainingsziel und/oder die zur Verfügung stehenden Zeitspanne für das Training usw. eingehen kann. Wird weiter der Belastungsbereich für das Training an diesem Tag aus, dem zu Trainierenden zuvor individuell vorgegebenen, unterschiedlichen Belastungsbreichen ausgewählt und zum Training empfohlen, kann das Verfahren noch verbessert und zusätzlich auch besonders einfach und zuverlässig gestaltet werden.

Ein wesentlicher erfindungsgemäßer Vorteil ist, dass der ausgewählte Belastungsbereich unter Berücksichtigung zuvor individuell festgelegter Parameter erfolgt. Es ist auf diese Weise etwa möglich, dass der zu Trainierende z.B. selbst bei einem besonders guten aktuellen physischen und/oder psychischen Zustand an diesem Tag nicht außerhalb der für Ihn vorab individuell vorgegebenen Grenzen - etwa hinsichtlich einer Belastungsintensität - trainiert. So kann durch die Erfindung unter anderem ein besonders sicheres und einfaches Verfahren zur Verfügung gestellt werden. Letzteres dadurch, dass zuvor individuell vorgegebene Belastungsbereiche vorliegen, diese also beispielsweise nicht stets neu festgelegt, dargestellt etc. werden. Somit kann ein für ein bevorstehendes Training ausgewählter Belastungsbereich vom zu Trainierenden auch erleichtert erfasst, zugeordnet und umgesetzt werden - etwa, da Ihm dieser hinsichtlich der Belastungsintensität, Art der Belastung oder Ähnlichem bereits bekannt sein können. Unter anderem kann aber auch sichergestellt werden, dass eine Trainingsempfehlung gewählt wird, die mit erhöhter Wahrscheinlichkeit oder in erhöhtem Ausmaß, z.B. zur Erreichung eines gewünschten Trainingsziels beiträgt, womit die Effektivität des Verfahrens erhöht wird. Dennoch ist, da aus unterschiedlichen Belastungsbereichen ausgewählt werden kann, eine vergleichsweise hohe Variabilität gewährleistet - wobei beispielsweise auch noch zusätzlich eine Abstimmung von Belastungsintensität oder -intensitäten mit der Dauer des Trainings erfolgen kann.

Erfindungsgemäß kann also eine vorteilhafte Trainingsempfehlung gegeben werden - zum einen sind unterschiedliche Belastungsbereiche individuell vorgegeben und tragen somit als eine Art Grundlage für Verfahren zum Erzeugen einer Anweisung bei, zum anderen findet jedoch der aktuelle physische und/oder psychische Zustand besondere Berücksichtigung - womit diese Faktoren besonders gut aufeinander abgestimmt werden.

Es wird festgehalten, dass der zu Trainierende sowohl ein Mensch, als auch ein Tier sein kann. Insbesondere bei Pferden kann sich dieses Verfahren bewähren.

Im Allgemeinen wird festgehalten, dass sich die Bestimmung des physischen und/ oder psychischen Zustands des zu Trainierenden anhand seiner Herzratenvariabilität als besonders zuverlässig herausgestellt hat. Dies kann aber auch anhand der Herzfrequenz (HF) gelingen. Als vorteilhaft kann sich erweisen, wenn die Herzratenvariabilität und auch die Herzfrequenz gemeinsam betrachtet werden. Im Allgemeinen wird erwähnt, dass zur Analyse der Herzratenvariabilität eine Vielzahl von Verfahren zum Einsatz kommen können. Insbesondere haben sich zur Ermittlung des physischen und/oder psychischen Zustands Analysen im Zeitbereich bewährt. Hierbei werden über einen bestimmten Zeitraum die Schwankungen der Dauer von Interbeat-Intervallen (IBI), insbesondere von RR-Intervallen analysiert und aus jener Schwankung die Herzratenvariabilität errechnet - auf deren Basis ist es möglich, besonders zuverlässige und genaue Rückschlüsse auf den physischen und/oder psychischen Zustand eines Probanden zu ziehen. Als insbesondere vorteilhaft haben sich in diesem Zusammenhang jene Verfahren herausgestellt, bei denen die Analyse der Herzratenvariabilität eine Untersuchung wenigstens einer Häufigkeitsverteilung auf eine multimodale Verteilung hin umfasst.

Im Allgemeinen wird festgehalten, dass Belastungsbereiche als Bereiche von unterschiedlichen körperlichen Belastungsintensitäten zu verstehen sind. Ein Belastungsbereich umfasst also eine gewisse Bandbreite an Belastungsintensitäten. Diese können etwa durch die physischen Reaktionen des Trainierenden, insbesondere durch dessen Herzfrequenzen beim Training, definiert werden - vorstellbar sind als Belastungsbereiche etwa prozentuale Bereiche der maximalen Herzfrequenz ($HF_{max}$) des Trainierenden. Die maximale Herzfrequenz kann durch verschiedene Verfahren bestimmt werden, bekannt sind auch diverse Formeln, anhand derer auf die maximale Herzfrequenz des Trainierenden in Abhängigkeit unterschiedlicher physischer Kennzahlen geschlossen wird. Laut einer dieser Formeln kann die maximale Herzfrequenz etwa anhand

$$HF_{max} = 220 - Alter \text{ (in Jahren)}$$

errechnet werden. Eine auf diese Art ermittelte maximale Herzfrequenz kann jedoch nur eine grobe Abschätzung liefern und eignet sich im Allgemeinen nicht zur Bestimmung der Belastungsbereiche bei Trainierenden mit hoher Leistungsfähigkeit. Im Allgemeinen wird weiter festgehalten, dass die Belastungsbereiche auch so definiert werden können, dass durch diese zielgerichtet eine bestimmte oder gewünschte körperliche Entwicklung erfolgen kann. Hierbei werden die Belastungsbereiche so gegeneinander abgegrenzt, dass Belastungen entsprechend den Belastungsintensitäten des Belastungsbereichs zu unterschiedlichen physischen Anpassungen des trainierten Körpers führen. Dies könnte zum Beispiel der Belastungsbereich Grundlagenausdauer zur Entwicklung der Grundlagenausdauerfähigkeit sein. Die Grenzen der Belastungsbereiche können hierbei etwa als prozentuale Werte der maximalen Herzfrequenz angegeben werden, oder direkt aus einem Leistungstest ermittelt werden. Im Allgemeinen wird weiter festgehalten, dass für Sportarten unterschiedliche Belastungsbereiche vorgegeben werden können. Dies kann insbesondere dann von Vorteil sein, wenn die maximale Herzfrequenz des Trainierenden zwischen den Sportarten variiert.

Werden die individuell vorgegebenen Belastungsbereiche in Abhängigkeit wenigstens zweier, an vorangegangenen Tagen bestimmten physischen und/oder psychischen Zuständen anhand von an diesen Tagen erfassten Herzratenvariabilität (HRV) festgelegt, kann das erfindungsgemäße Verfahren weiter verbessert werden. Dadurch ist es nämlich insbesondere möglich, einen längerfristigen physischen und/oder psychischen Status des zu Trainierenden zu berücksichtigen. Dies stellt sich nicht zuletzt deshalb als Vorteil heraus, weil etwa diesbezügliche natürliche Schwankungen - bedingt durch wechselnde Ernährungsgewohnheiten, Schlafqualität usw. -, verringert werden können. Auch kann auf diese Weise die Veränderung des physischen und/oder psychischen Zustands des zu Trainierenden, nicht zuletzt aufgrund seines Trainings, verbessert Eingang in das Verfahren finden - womit in weiterer Folge auch der, aus den zuvor individuell vorgegebenen Belastungsbreichen ausgewählte Belastungsbereich dem aktuellen Zustand des zu Trainierenden verbessert entspricht. Besonders die Berücksichtigung der Herzratenvariabilität kann sich in diesem Zusammenhang als Vorteil erweisen. Diese erweist sich nämlich als äußerst zuverlässig im Erkennen von physischen und/oder psychischen Zuständen eines zu Trainierenden. So kann beispielsweise ein Übertraining, also eine chronische Überlastungsreaktion, durch die Bestimmung der Herzratenvariabilität besonders gut erkannt werden. Somit ist also noch besser sicherstellbar, dass die individuell vorgegebenen unterschiedlichen Belastungsbereiche auf den zu Trainierenden besonders gut abgestimmt sind. Die genannten Vorteile etwa hinsichtlich Sicherheit des Verfahrens, Effektivität zur Erreichung eines Trainingsziels usw. sind somit noch weiter verbessert. In besonderem Ausmaß kann sich dies hinsichtlich Messungenauigkeiten, natürlichen Schwankungen etc. bei der Bestimmung des aktuellen psychischen und/oder physischen Zustands als Vorteil herausstellen.

Zuvor Genanntes kann auch verbessert erreicht werden, wenn die individuell vorgegebenen Belastungsbereiche in regelmäßigen zeitlichen Intervallen oder entsprechend der Häufigkeit des Trainings neu vorgegeben werden. So kann sichergestellt werden, dass die Anweisung zu einem bevorstehenden Training auf Belastungsbereichen basiert, die dem Trainingszustand des Trainierenden besonders gut entsprechen. Es ist insbesondere vermeidbar, dass zu niedrige Belastungsintensitäten empfohlen werden, obwohl aufgrund eines verbesserten Trainingszustands des Trainierenden höhere zur Erzielung des Trainingseffekts erforderlich wären - in diesem Fall würden nämlich keine Belastungsbereiche mit derart niedrigen Belastungsintensitäten zur Verfügung stehen, aus denen ausgewählt werden kann. Ebenso kann vermieden werden, dass dem Trainierenden zu hohe Belastungsintensitäten empfohlen werden, wenn der Trainingszustand des Trainierenden, z.B. aufgrund einer verletzungsbedingten Pause, niedrigere Belastungsintensitäten bedingen würde. Insbesondere erweist sich in diesem Zusammenhängen als vorteilhaft, die Belastungsbereiche monatlich, entsprechend der Häufigkeit des Trainings - und somit abgestimmt auf die zu erwartenden Änderungen hinsichtlich des Verfassung des zu Trainierenden - neu zu vergeben. Insbesondere erweist es sich als vorteilhaft, die Belastungsbereiche quartalsweise neu zu vergeben. Die Anweisungen können demnach in Hinblick auf das zu erreichenden Trainingsziel zuverlässiger gestaltet werden.

Werden die individuell vorgegebenen Belastungsbereiche des zu Trainierenden in Abhängigkeit von Referenzdaten anhand seines Alters und/oder Geschlechts und/oder anhand seines gesundheitlichen Zustands festgelegt, können Belastungsbereiche, aber auch Belastungsintensitäten besonders gut an die gegebenen Voraussetzungen sowie an die zu erreichenden Ziele abgestimmt werden. Dies gilt insbesondere, wenn neben derartigen Referenzdaten auch Zustände anhand der erfassten Herzratenvariabilität (HRV) und/oder Herzfrequenz (HF) einfließen.

Umfassend die individuell vorgegebenen Belastungsbereiche zumindest einen der Bereiche Ruhebereich, Regeneration und Fettverbrennung (RG/FV), Grundlagenausdauer 1 (GA1), Grundlagenausdauer 2 (GA2), Entwicklungsbereich (EB) und Spitzenbereich (SB), ist es möglich, wesentliche Belastungsbereiche schnell und einfach für das Verfahren zu berücksichtigen. Zudem sind die Belastungsintensitäten auf diese Weise einfach zu definieren und zu vermitteln, sodass diese vom zu Trainierenden leicht erfass-, zuorden- und durchführbar sind. Des Weiteren können körperliche Aktivitäten eines zu Trainierenden rasch nahezu überschneidungsfrei klassifiziert und/oder hinsichtlich ihrer Belastungsintensität zugeordnet werden. Im Allgemeinen kann sich als vorteilhaft herausgestellt, wenn eine individuelle Festlegung diese Bereiche unter Berücksichtigung der Herzratenvariabilität erfolgt.

Werden die individuell vorgegebenen Belastungsbereiche in Abhängigkeit eines Leistungstests festgelegt, kann der Trainierende - z.B. in zeitlicher Hinsicht - vergleichsweise unabhängig seine aktuelle Leistungsfähigkeit feststellen und seine Belastungsbereiche aktualisieren. Dadurch ist es möglich, das Verfahren nicht nur benutzerfreundlicher, sondern auch zuverlässiger zu gestalten, da beispielsweise Änderungen der Leistungsfähigkeit des zu Trainierenden schneller berücksichtigt werden können. Eine zuverlässigere Vorgabe der Belastungsbereiche ist auf Basis verschiedener Leistungstests möglich. So können etwa über einen Laktattest, Spinergometrie oder durch Bestimmung der maximalen Sauerstoffaufnahme genau auf den Trainingszustand des Trainierenden abgestimmte Belastungsbereiche vorgegeben werden.

Wird mindestens ein Belastungsbereich aus einem Trainingsplan für mehrere Tage, der die individuell vorgegebenen Belastungsbereiche aufweist, ausgewählt, so kann das erfindungsgemäße Verfahren nicht nur besonders gut gesteuert, sondern auch benutzerfreundlicher gestaltet werden. Auf diese Weise ist unter anderem eine verbesserte Vorhersehbarkeit der Trainings für den zu Trainierenden möglich. So kann vorgesehen sein, dass ein Trainingsplan für eine gewisse Anzahl von Tagen festgelegt wird, der ebenso viele individuell vorgegebene Belastungsbereiche oder Kombinationen von diesen aufweist. Abhängig vom aktuellen physischen und/oder psychischen Zustand wird dann einer der vorgegebenen Belastungsbereiche oder eine Kombination für das Training eines Tages ausgewählt. Eine zusätzliche Feinabstimmung des Trainings dieses Tages hinsichtlich des aktuellen Zustands des zu Trainierenden kann erfolgen, indem das Verfahren z.B. die Belastungsdauer innerhalb dieses Belastungsbereichs, die Belastungsintensität und/oder die Art der Belastung etc. entsprechend festlegt. Somit ist dem zu Trainierenden unter anderem bekannt, welche Belastungsbereiche innerhalb der Dauer des Trainingsplans geplant bzw. in dessen Verlauf noch bevorstehen - womit die Benutzerfreundlichkeit aber auch die Motivation erhöht werden können. Unter anderem kann nämlich vorab verbessert vermittelt werden, welche Belastungsbereiche innerhalb des Trainingsplans noch bevorstehen - womit sich der zu Trainierende in Bezug auf die erforderliche Zeitdauer usw. bereits vorab verbessert einstellen kann. Aber auch der Trainingsplan kann verbessert auf Gegebenheiten und Möglichkeiten des zu Trainierenden verbessert abgestimmt werden - etwa indem vorab festgelegt wird, dass innerhalb der Dauer eines Trainingsplans lediglich einzelne Tage für ein Training zur Verfügung stehen. In diesem Fall ist vorstellbar, die zuvor individuell vorgegebenen Trainingsbereiche vorausschauend zu adaptieren. Die Trainingsempfehlungen können so zielgerichteter und das Verfahren verlässlicher gestaltet werden. Es ist also gezeigt, dass erfindungsgemäß ein Verfahren mit besonders hoher Einfachheit und Benutzerfreundlichkeit für optimierte Trainingsergebnisse erreicht wird.

Wird eine Trainingsart entsprechend dem ausgewählten Belastungsbereich für das Training an diesem Tag empfohlen, kann gewährleistet werden, dass das Verfahren für den zu Trainierenden besonders einfach, benutzerfreundlich bzw. sicher ist. Dadurch kann unter anderem verbessert erreicht werden, dass der Trainierende in optimaler Intensität, Dauer, etc. trainiert, womit die Effektivität und Steuerbarkeit des Trainings erhöht werden.

Werden mehrere ausgewählte Belastungsbereiche anhand des aktuellen physischen und/oder psychischen Zustands des zu Trainierenden zueinander gewichtet und zum Training empfohlen, können die Steuerbarkeit und die Feinabstimmung des bevorstehenden Trainings, aber auch die Variabilität der Anweisung weiter erhöht werden. Auf diese Weise kann eine individuelle und auf den aktuellen Zustand des zu Trainierenden verbessert abgestimmte Empfehlung abgegeben werden, die für den zu Trainierenden dennoch leicht zu erfassen ist und mit deren Hilfe in Abhängigkeit seiner Tagesverfassung die optimale Belastungsintensität, Belastungsdauer, etc., oder eine Kombination verschiedener Belastungsintensitäten ausgewählt werden können.

In den Figuren ist beispielsweise der Erfindungsgegenstand anhand einer Ausführungsvariante näher dargestellt. Es zeigen

Fig. 1     eine beispielhafte grafische Benutzeroberfläche (GUI) eines Computerprogramms, darstellend den Regenerationsstatus des Trainierenden und eine Trainingsempfehlung für das nächste Training,

Fig. 2     eine beispielhafte grafische Benutzeroberfläche (GUI) eines Computerprogramms, darstellend eine gewichtete Trainingsempfehlung für das nächste Training,

Fig. 3     eine beispielhafte grafische Benutzeroberfläche (GUI) eines Computerprogramms, darstellend den aktuellen Status des laufenden Trainings,

Fig. 4     eine grafische Darstellung der Einteilung der Belastungsintensitäten und Herzraten in Belastungsbereiche, und

Fig. 5     eine grafische Darstellung eines Trainingsplans.

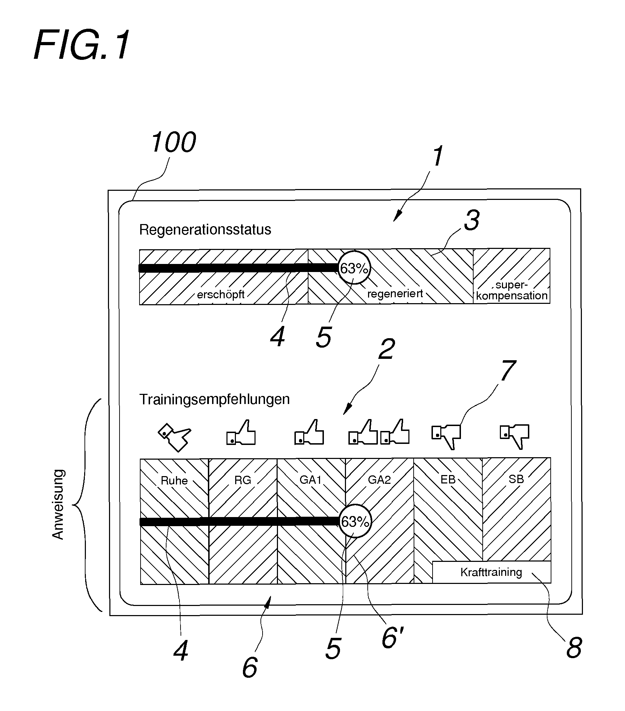

Gemäß Fig. 1 wird beispielhaft eine grafische Benutzeroberfläche 100 eines Computerprogramms zum Erzeugen einer Anweisung für ein körperliches Training eines Menschen entsprechend einer Ausführungsart der Erfindung gezeigt. Wie bereits erwähnt, ist das erfindungsgemäße Verfahren jedoch nicht auf Personen eingeschränkt, vielmehr kann es sich auch bei Tieren bewähren. Hierbei ist der physische und/oder psychische Zustand des zu Trainierenden in Form des Regenerationsstatus 1 dargestellt. Zur Bestimmung des Regenerationsstatus 1 wird die Herzratenvariabilität (HRV) des zu Trainierenden erfasst, allerdings ist dies auch anhand dessen Herzfrequenz möglich. In diesem Ausführungsbeispiel wird der Regenerationsstatus 1 in verschiedene Regenerationsstufen 3 unterteilt. Ein grafischer Indikator 4 visualisiert den Regenerationsstatus 1 entsprechend den Regenerationsstufen 3 sowie anhand eines numerischen Regenerationswerts 5, der den Regenerationsstatus 1 zusätzlich quantifiziert. Zusammen mit einem grafischen Indikator 4, zeigt der numerische Regenerationswert 5 jene Regenerationsstufe 3 an, welche dem aktuellen Regenerationsstatus 1 des Trainierenden entspricht.

Erfindungsgemäß wird nun anhand des am Tag des Trainings bestimmten aktuellen physischen und/oder psychischen Zustands ein Belastungsbereich 6' für das Training an diesem Tag ausgewählt. Somit können die Vorteile aufgrund der Abstimmung des Trainings an einem Tag auf den aktuell an diesem Tag vorliegenden Status des zu Trainierenden erreicht und das bevorstehende Training optimiert werden. So können also etwa die Auswirkungen eines vorangegangenen körperlichen Trainings oder der Lebensumstände des zu Trainierenden verbessert berücksichtigt werden.

Zudem wird der Belastungsbereich 6' aus, dem zu Trainierenden zuvor individuell vorgegebenen Belastungsbreichen 6 ausgewählt und zum Training empfohlen. Hierzu zeigt die untere Hälfte der Fig. 1 beispielhaft eine Anweisung zu einem bevorstehenden Training, umfassend eine Trainingsempfehlung 2. Es wird in diesem Ausführungsbeispiel zudem eine Empfehlung 2 zu mehreren Belastungsbereichen 6 gezeigt. Da diese Belastungsbereiche 6 dem zu Trainierenden zuvor individuell vorgegeben wurden, kann im Gegensatz zum Stand der Technik nun ein besonders sicheres und zuverlässiges Verfahren zur Verfügung gestellt werden. So können die Belastungsbereiche nämlich nicht nur einfach - etwa entsprechend dem Alter des zu Trainierenden und/oder dessen gewünschten Trainingseffekten oder -zeiten und/oder entsprechend HRV-Messungen an vorangegangenen Tagen usw. - vorab festgelegt werden. Zudem wird verbessert vermieden, dass Belastungsgrenzen unerwünscht überschritten werden, etwa aufgrund eines Ausreißers bei der Bestimmung des aktuellen physischen und/oder psychischen Zustands des zu Trainierenden. Zudem ist eine einfache und sichere Vermittlung der Belastungsbereiche für den zu Trainierenden möglich womit dieser die Trainingsempfehlungen 2 schneller auffassen kann - unter anderem, da diese ja vorgegeben sind. Um den ausgewählten Belastungsbereich 6' benutzerfreundlich anzuzeigen, werden die Belastungsbereiche 6 hierfür mit dem grafischen Indikator 4 des Regenerationsstatus 1 und mit dem numerischen Regenerationswert 5 überlagert, wobei der numerische Regenerationswert 5 als Zeiger für den grafischen Indikator 4 fungiert und jenen Belastungsbereich 6' markiert, welcher für das bevorstehende Training besonders empfohlen wird.

Als Belastungsbereiche 6 werden in diesem Ausführungsbeispiel durch Ruhebereich, die Regeneration und Fettverbrennung (RG/FV), die extensive Grundlagenausdauer 1 (GA1), die intensive Grundlagenausdauer 2 (GA2), den Entwicklungsbereich (EB) und den Spitzenbereich (SB) dargestellt. Die Erfindung beschränkt sich jedoch im Allgemeinen nicht auf die Auswahl dieser speziellen Belastungsbereiche 6. Vielmehr können sich die Belastungsbereiche 6 hinsichtlich der gewünschten/individuell optimalen Trainingsart unterscheiden - oder auch in Abhängigkeit von Referenzwerten anhand seines Alters, Geschlechts, seines gesundheitlichen Zustands und/oder seiner an vorangegangenen Tagen bestimmten HRV-Werte festgelegt werden.

In der Figur 1 wird weiter gezeigt, dass eine Trainingsempfehlung 2 zu mehreren Belastungsbereichen 6 gegeben wird - nämlich anhand der, den jeweiligen Belastungsbereichen 6 zugeordneten grafischen Indikatoren 7, welche die Eignung des jeweiligen Belastungsbereichs 6 für das bevorstehende Training in mehreren Abstufungen - zwischen empfohlen und nicht empfohlen - auf einfache Weise erkennbar darstellen. Im vorliegenden Beispiel wäre also eine vereinfachte Darstellung der Empfehlung für den zu Trainierenden, vornehmlich im Bereich GA2 zu trainieren. Zusätzlich wird eine Information 8 zur Trainingsart einzelner angeführter Belastungsbereiche abgegeben - in der Figur 1 fällt Krafttraining in die jeweiligen Belastungsbereiche, selbstverständlich können Informationen zu Trainingsarten anderen Belastungsbereiche ebenso vorgesehen sein.

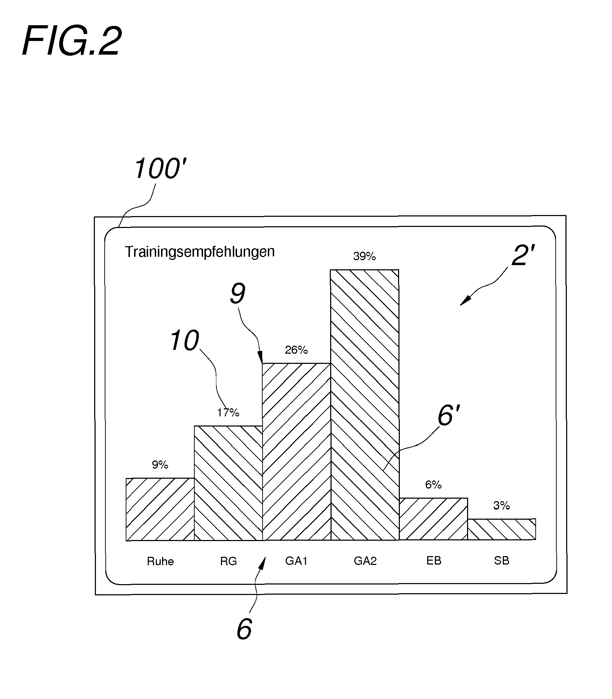

Dadurch kann ein besonders gut abgestimmtes Training ermöglicht werden. Dies wird in Fig. 2 gemäß einer weiteren Darstellung einer grafischen Benutzeroberfläche 100' eines Computerprogramms gezeigt, welche eine gewichtete Trainingsempfehlung 2' für das nächste Training aufweist. Hierzu werden mehrere Belastungsbereichen aus den Belastungsbereichen 6 ausgewählt und anhand des aktuellen physischen und/oder psychischen Zustands des zu Trainierenden zueinander gewichtet und zum Training empfohlen. Dies kann sich insbesondere bewähren, um ein Training abwechslungsreicher zu gestalten oder auch, um besonders gut auf den Zustand des zu Trainierenden einzugehen - etwa wenn der aktuelle psychische und/oder physische Zustand in einem Grenzbereich zweier der vorgegebenen Belastungsbereiche 6 auswählen ließe. In der exemplarisch dargestellten grafischen Benutzeroberfläche 100', wird die gewichtete Trainingsempfehlung 2' als Balkendiagramm 9 mit entsprechenden Gewichtungsfaktoren 10 gezeigt. Eine derartige gewichtete Trainingsempfehlung kann auch entsprechend Fig. 1 dargestellt werden. Die grafischen Indikatoren 7 ersetzen hierbei das Balkendiagramm 9 aus Fig. 2, und geben eine gewichtete Trainingsempfehlung für jeden Belastungsbereich 6 an. Solch eine gewichtete Trainingsempfehlung ist insbesondere vorteilhaft, da sie vom Trainierenden visuell leicht erfasst werden kann und dieser flexibel auf die Trainingsempfehlung reagieren kann. So kann der Trainierende seiner eigenen Empfindung nach einen Belastungsbereich entsprechend der gewichteten Trainingsempfehlung wählen, welcher am besten seiner Tagesverfassung entspricht.

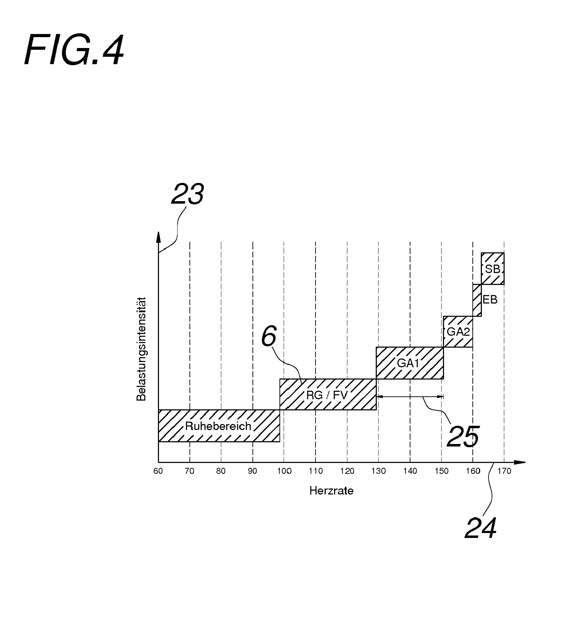

Gemäß Fig. 4 wird eine grafische Darstellung der Einteilung der Belastungsintensitäten 23 und Herzraten 24 in Belastungsbereiche 6, die lediglich der einfacheren Darstellung wegen in Form von Balken dargestellt sind, gezeigt. Die Belastungsbereiche 6 von körperlichen Belastungsintensitäten sind in diesem Ausführungsbeispiel durch Herzratenbereiche definiert. So zeigt die Figur 4 Belastungsbereiche 6 mit unterschiedlichen körperlichen Belastungsintensitäten - etwa der Belastungsbereich GA1 innerhalb der Herzratenbereiche 25. Die Herzratenbereiche können sich wahlweise überschneiden, oder voneinander separiert sein. Ein vollständiger Satz der Belastungsbereiche 6 deckt im vorliegenden Beispiel die Herzraten 24 möglichst umfassend ab. Der Vollständigkeit halber wird weiter erwähnt, dass die Belastungsbereiche neben der Herzrate auch als Bereiche anderer Messgrößen definiert werden können. So ist beispielsweise vorstellbar, dass die Belastungsbereiche als Laufgeschwindigkeitsbereiche, Radfahrgeschwindigkeitsbereiche, Trittfrequenzbereiche, Leistungsbereiche (z. B. in Watt) etc. ausgeführt sein können - es ist also möglich, die Belastungsbereiche auch in Abhängigkeit der Trainingsarten, etwa Laufen, Schwimmen etc. festzulegen. Ein Belastungsbereich 6 kann zudem über Parameter wie bestimmte Laktatwerte im Blut usw. definiert werden.

In diesem Zusammenhang bewährt sich, die individuell vorgegebenen Belastungsbereiche in Abhängigkeit wenigstens zweier, an vorangegangenen Tagen bestimmten physischen und/oder psychischen Zuständen in Abhängigkeit der erfassten Herzratenvariabilität (HRV) festzulegen. Es ist zudem vorteilhaft, die individuell vorgegebenen Belastungsbereiche 6 in regelmäßigen zeitlichen Intervallen, insbesondere quartalsweise, oder entsprechend der Häufigkeit des Trainings neu vorzugeben. Ebenso ist vorstellbar, dass die Belastungsbereiche 6 durch einen Trainer oder bei einer sportärztlichen Untersuchung für den Trainierenden neu vorgegeben werden. Als besonders einfach und vorteilhaft kann sich jedoch erweisen, wenn der Trainierende selbst die Belastungsbereiche 6 durch einen Leistungstest auf seine aktuelle Leistungsfähigkeit abstimmt.

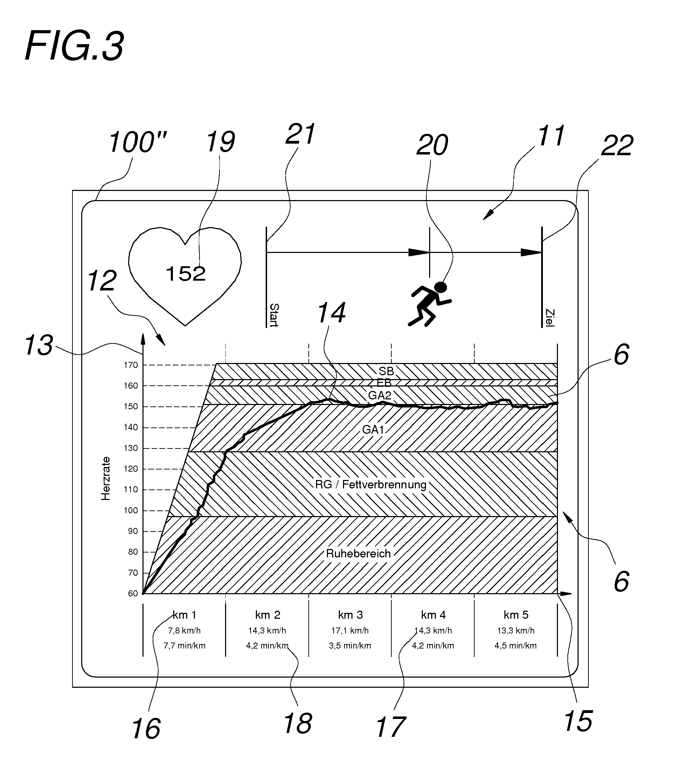

Gemäß Fig. 3 wird beispielhaft eine grafische Benutzeroberfläche 100" eines Computerprogramms, entsprechend einer Ausführungsart der Erfindung, gezeigt, welche den aktuellen Trainingsstatus 11 eines laufenden Trainings darstellt. Hierbei wird in einem Diagramm 12 die Herzrate 13 in Form einer Herzratenkurve 14 als Funktion des Trainingsfortschritts 15 dargestellt. Im vorliegenden Beispiel wird der Trainingsfortschritt 15 durch die zurückgelegte Entfernung 16 repräsentiert. Die Herzratenkurve 14 wird im Diagramm 12 zusätzlich mit den Belastungsbereichen 6 überlagert, wodurch einfach visuell erkennbar der momentane Belastungsbereich und die Belastungsbereiche 6 zu jedem früheren Zeitpunkt dargestellt werden. Zusätzlich werden weitere Daten des laufenden Trainings, wie die Geschwindigkeit 17 und die Zeit pro Kilometer 18, dargestellt. In einem Bereich der grafischen Benutzeroberfläche 100" wird die momentane Herzrate 19 angezeigt. Der Trainingsfortschritt 15 wird zusätzlich mit einem grafischen Indikator 20, welcher sich mit fortschreitendem Trainingsfortschritt 15 ausgehend von Start 21 nach Ziel 22 bewegt. Der Vollständigkeit halber wird erwähnt, dass der Trainingsfortschritt 13 auf verschiedenste Weise, etwa auch durch die verstrichene Trainingsdauer, durch eine verbrauchte Kalorienanzahl oder durch die Anzahl der gemachten Schritte dargestellt werden kann.

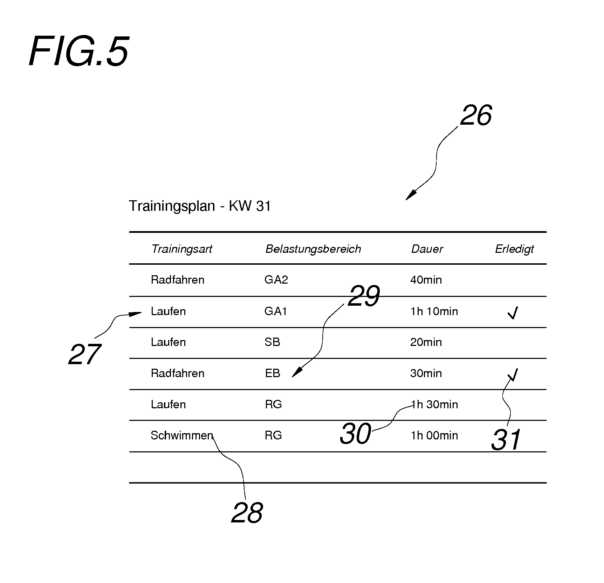

Gemäß Fig. 5 wird ein Beispiel zu einem Trainingsplan 26 für eine Woche gezeigt. Dieser Trainingsplan 26 umfasst sieben individuell vorgegebene Belastungsbereiche 29, wobei auch mehrere Belastungsbereiche für einen Tag vorstellbar sind. Abhängig vom aktuellen physischen und/oder psychischen Zustand wird einer oder auch mehrere der vorgegebenen Belastungsbereiche 29, die in dieser Variante über die Herzrate definiert sind, für das Training eines Tages ausgewählt. Des Weiteren wird auch eine Trainingsart 28 und eine Trainingsdauer 30 zu dem ausgewählten Belastungsbereich 29 empfohlen, sodass die entsprechenden Parameter einer Trainingseinheit 27 festgelegt sind. Auf diese Weise wird nicht nur eine hohe Variabilität des Trainings, sondern auch eine besonders gute Feinabstimmung hinsichtlich des aktuellen Zustands des zu Trainierenden ermöglicht. Das Training ist also auch besonders sicher, da selbst bei einem Ausreißer oder einer fehlerhaften Bestimmung des aktuellen physischen und/oder psychischen Zustands nur aus individuell vorgegebenen Belastungsbereichen ausgewählt wird. Das Verfahren zeigt sich auch besonders zuverlässig und gut steuerbar - ebenso ist die Benutzerfreundlichkeit für den zu Trainierenden erhöht. Es ist vorstellbar, dass die Trainingsdauern 30 oder auch die Trainingsart 28 im Trainingsplan entsprechend dem aktuellen physischen und oder psychischen Zustand des zu Trainierenden adaptiert werden. Für Trainingseinsteiger kann es vorteilhaft sein, Trainingspläne über weniger Tage zu erstellen, da die Auswirkungen eines vorangegangenen Trainings vielfach nur schwer vorhersehbar sind und die zuvor individuell vorgegebenen unterschiedlichen Belastungsbereiche ebenfalls häufiger neu vorzugeben sind. Zusätzlich kann eine Markierung 31 vorgesehen sein, welche angibt, ob eine Trainingseinheit 27 bereits erledigt wurde. Es wird also gezeigt, dass das erfindungsgemäße Verfahren besonders gut an die gegebenen körperlichen und Psychischen Voraussetzungen, sportlichen Ziele etc. des zu Trainierenden abgestimmt ist. Aber auch eine besonders gute Berücksichtigung des aktuellen körperlichen und psychischen Zustands aufgrund vorangegangener Trainingseinheiten und auch der Entwicklung der Leistungsfähigkeit aufgrund dieser sind ermöglicht.

**Patentansprüche**

1.  Verfahren zum Erzeugen einer Anweisung für ein körperliches Training, bei dem
    die Herzratenvariabilität (HRV) und/oder die Herzfrequenz (HF) des zu Trainierenden erfasst und in deren Abhängigkeit dessen physischer und/oder psychischer Zustand (1) bestimmt und
    anhand dieses Zustands für diesen eine Anweisung zu einem bevorstehenden Training erzeugt wird, die eine Empfehlung zu mindestens einem Belastungsbereich (6) von körperlichen Belastungsintensitäten umfasst,
    **dadurch gekennzeichnet, dass**
    anhand des am Tag des Trainings bestimmten aktuellen physischen und/oder psychischen Zustands mindestens ein Belastungsbereich (6') für das Training an diesem Tag aus, dem zu Trainierenden zuvor individuell vorgegebenen, unterschiedlichen Belastungsbreichen (6) ausgewählt und zum Training empfohlen wird.

2.  Verfahren nach Anspruch 1, **dadurch gekennzeichnet, dass** die individuell vorgegebenen Belastungsbereiche (6) in Abhängigkeit wenigstens zweier, an vorangegangenen Tagen bestimmten physischen und/oder psychischen Zuständen anhand von an diesen Tagen erfassten Herzratenvariabilität (HRV) festgelegt werden.

3.  Verfahren nach Ansprüche 1 oder 2, **dadurch gekennzeichnet, dass** die individuell vorgegebenen Belastungsbereiche (6) in regelmäßigen zeitlichen Intervallen, insbesondere monatlich, entsprechend der Häufigkeit des Trainings, insbesondere quartalsweise, neu vorgegeben werden.

4.  Verfahren nach Anspruch 1, 2 oder 3, **dadurch gekennzeichnet, dass** die individuell vorgegebenen Belastungsbereiche (6) des zu Trainierenden in Abhängigkeit von Referenzdaten anhand seines Alters und/oder Geschlechts und/oder anhand seines gesundheitlichen Zustands festgelegt werden.

5. Verfahren nach einem der Ansprüche 1 bis 4, **dadurch gekennzeichnet, dass** die individuell vorgegebenen Belastungsbereiche (6) zumindest einen der Bereiche Ruhebereich, Regeneration und Fettverbrennung (RG/FV), Grundlagenausdauer 1 (GA1), Grundlagenausdauer 2 (GA2), Entwicklungsbereich (EB) und Spitzenbereich (SB) umfassen.

6. Verfahren nach einem der Ansprüche 1 bis 5, **dadurch gekennzeichnet, dass** die individuell vorgegebenen Belastungsbereiche (6) in Abhängigkeit eines Leistungstests festgelegt werden.

7. Verfahren nach einem der Ansprüche 1 bis 6, **dadurch gekennzeichnet, dass** mindestens ein Belastungsbereich aus einem Trainingsplan (26) für mehrere Tage, der die individuell vorgegebenen Belastungsbereiche (29) aufweist, ausgewählt wird.

8. Verfahren nach einem der Ansprüche 1 bis 7, **dadurch gekennzeichnet, dass** eine Trainingsart (28) entsprechend dem ausgewählten Belastungsbereich für das Training an diesem Tag empfohlen wird.

9. Verfahren nach einem der Ansprüche 1 bis 8, **dadurch gekennzeichnet, dass** mehrere ausgewählte Belastungsbereiche anhand des aktuellen physischen und/oder psychischen Zustands des zu Trainierenden zueinander gewichtet und zum Training empfohlen werden.

# FIG.1

# FIG.2

100'

Trainingsempfehlungen 39% 2'

9

10

26%

17%

9%

6'

6%

3%

Ruhe          RG          GA1          GA2          EB          SB

6

# FIG.3

SB
EB
GA2
GA1
RG / Fettverbrennung
Ruhebereich

Herzrate

170
160
150
140
130
120
110
100
90
80
70
60

152

Start
Ziel

| km 1 | km 2 | km 3 | km 4 | km 5 |
|---|---|---|---|---|
| 7,8 km/h | 14,3 km/h | 17,1 km/h | 14,3 km/h | 13,3 km/h |
| 7,7 min/km | 4,2 min/km | 3,5 min/km | 4,2 min/km | 4,5 min/km |

# FIG.4

# FIG.5

*26*

Trainingsplan - KW 31

| Trainingsart | Belastungsbereich | Dauer | Erledigt |
|---|---|---|---|
| Radfahren | GA2 | 40min | |
| Laufen | GA1 | 1h 10min | ✓ |
| Laufen | SB | 20min | |
| Radfahren | EB | 30min | ✓ |
| Laufen | RG | 1h 30min | |
| Schwimmen | RG | 1h 00min | |

*29*

*27*

*30*

*31*

*28*

**Europäisches Patentamt**
**European Patent Office**
**Office européen des brevets**

## EUROPÄISCHER RECHERCHENBERICHT

**Nummer der Anmeldung**

EP 14 19 6420

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | ANTTI M KIVINIEMI ET AL: "Endurance training guided individually by daily heart rate variability measurements", EUROPEAN JOURNAL OF APPLIED PHYSIOLOGY, SPRINGER, BERLIN, DE, Bd. 101, Nr. 6, 12. September 2007 (2007-09-12), Seiten 743-751, XP019563432, ISSN: 1439-6327, DOI: 10.1007/S00421-007-0552-2 * das ganze Dokument * ----- | 1-9 | INV. G06F19/00 A63B24/00 |
| X | Craig Marker: "Heart Rate Variability: The New Science of Recovery", Breaking Muscle , 8. November 2014 (2014-11-08), XP002739478, Gefunden im Internet: URL:https://web.archive.org/web/20141108013824/http://breakingmuscle.com/strength-conditioning/heart-rate-variability-the-new-science-of-recovery [gefunden am 2015-05-11] * das ganze Dokument * ----- | 1-9 | |
| X,D | EP 1 852 062 A1 (POLAR ELECTRO OY [FI]) 7. November 2007 (2007-11-07) * Zusammenfassung * * Absätze [0002], [0006], [0007] * * Absätze [0035], [0042] - [0060] * * Abbildungen 5A, 5B * ----- | 1-9 | RECHERCHIERTE SACHGEBIETE (IPC)<br><br>G06F A63B |

Der vorliegende Recherchenbericht wurde für alle Patentansprüche erstellt

| Recherchenort | Abschlußdatum der Recherche | Prüfer |
|---|---|---|
| Den Haag | 12. Mai 2015 | Philips, Petra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 14 19 6420

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-05-2015

| Im Recherchenbericht angeführtes Patentdokument | Datum der Veröffentlichung | Mitglied(er) der Patentfamilie | Datum der Veröffentlichung |
|---|---|---|---|
| EP 1852062 A1 | 07-11-2007 | EP 1852062 A1<br>FI 20065286 A<br>US 2007287928 A1 | 07-11-2007<br>04-11-2007<br>13-12-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1852062 B1
- EP 2608090 A1

- EP 2260910 A1